# EUROPEAN PATENT SPECIFICATION

(11) **EP 1 196 151 B1**
(45) Date of publication and mention of the grant of the patent: **24.05.2006**
(21) Application number: 00945862.1
(22) Date of filing: 04.07.2000
(51) Int. Cl.: A61K 9/70, A61K 31/44, A61P 9/14

(54) **TRANSDERMAL THERAPEUTIC SYSTEM FOR ADMINISTERING A CALCIUM ANTAGONIST**
TRANSDERMALES THERAPEUTISCHES SYSTEM ZUR VERABREICHUNG VON CALCIUMANTAGONISTEN
SYSTEME THERAPEUTIQUE TRANSDERMIQUE POUR L'ADMINISTRATION D'UN ANTAGONISTE DU CALCIUM

(30) Priority: 22.07.1999 GB 9917290
(43) Date of publication of application: 17.04.2002
(73) Proprietor: LTS LOHMANN Therapie-Systeme AG, 56626 Andernach (DE)
(72) Inventor: BERTHOLD, Achim, D-56626 Andernach (DE); MÜLLER, Walter, D-56564 Neuwied (DE); GAVIRAGHI, Giovanni, Glaxo Wellcome S.p.A., I-37100 Verona (IT)
(74) Representative: Schmidt, Werner
(86) International application number: PCT/EP2000/006215
(87) International publication number: WO 2001/007017

(56) References cited:
- EP-A- 0 680 759
- WO-A-00/47208
- US-A- 4 956 171
- US-A- 4 983 395
- SHIRAKURA O; OHSHIMA A; TSUNEMI S: "Synergistic effect of D-Limonene and ethanol on the transdermal penetration of NB-818" DRUG DEVELOPMENT AND INDUSTRIAL PHARMACY, vol. 21, no. 4, 1995, pages 411-425, XP000961163

## Description

The present invention relates to a transdermal therapeutic system for the therapeutic administration of calcium antagonists of the dihydropyridine type, to a process for its preparation and to its use in medicine.

Calcium antagonists of the dihydropyridine type are compounds which influence the inflow of calcium ions into cells in particular into the cells of smooth muscles. Such compounds of the dihydropyridine type have been described, for example, in U.S. patent 3,799,934, U.S. patent 3,644,627, U.S. patent 4,264,611, and U.S. patent 4,801,599.

Calcium antagonists of the dihydropyridine type include, for example, amlodipine, felodipine, isradipine, lacidipine, nicardipine, nifedipine, nilvadipine, nimodipine, nisoldipine, and nitrendipine.

Diethyl (E)-4-[2-[(tert-butylcarbonyl)vinyl]phenyl-1,4-dihydro-2,6-dimethylpyridine-3,5 dicarboxylate (Lacidipine) is one of the preferred compounds of the dihydropyridine type. Lacidipine, which is described in British patent No. 2164336, is a potent long acting calcium antagonist which is particularly useful for treating hypertension. The compound may also be useful for the treatment of other cardiovascular disorders including atherosclerosis, peripheral vascular disease, ischaemic heart disease and congestive heart failure.

Nifedipine, which is described in U.S. patent 3,644,627, is another preferred calcium antagonists of the dihydropyridine type.

U.S. patent 4,983,395 pertains to transdermal drug delivery devices wherein the reservoir may comprise a gel consisting of nicardipine-hydrochloride, ® Klucel HF and a mixture of ethanol, water, glycerol and glycerol monooleate.

Transdermal drug delivery devices for co-administration of a drug such as nifedipine and a dual permeation enhancer comprising sucrose cocoate and methyl laurate are known from U.S. patent 4,956,171. The reservoir can contain nifedipine in methyl laurate or an aqueous solution of sucrose cocoate or in combination thereof.

EP-A 680,759 pertains to transdermal formulations of DHP calcium antagonists in a mixed liquid comprising cis-oleic acid and dimethylisosorbide dispersed in a propylene glycol base.

The promoting effect of a combination of limonene and ethanol has been found in Shirakura et al., Drug Development and Industrial Pharmacy, Vol. 21, No.4, 1995, pages 411 - 425 to synergistically enhance the transdermal adsorption of the DHP calcium antagonist NB-818.

Transdermal drug delivery systems provide a means for obtaining a high degree of control of drug concentration in the blood over a specified time period. Many systems have been developed and used to deliver drugs transdermally. It is however widely recognised that in general it is not possible to predict which particular systems will provide a satisfactory delivery system with a specific drug substance if that has not previously been adminstered by that route.

We have now found that calcium antagonists of the dihydropyridine type may be advantageously administered transdermally from a drug reservoir containing a solution comprising a calcium antagonist of the dihydropyridine type and at least one skin permeation enhancer.

Thus in one aspect the present invention provides a transdermal therapeutic system (hereinafter TTS) for administering calcium antagonists of the dihydropyridine type which comprises (a) a backing layer, which defines the upper surface of the device (b) a drug reservoir containing a solution comprising a calcium antagonist of the dihydropyridine type and at least one skin permeation enhancer, (c) a membrane to control the release of the active ingredient, (d) a pressure sensitive adhesive layer for attaching the system to the skin and, if necessary, a release liner on the outer face of the adhesive layer wherein the said backing layer and said membrane are connected together to form the drug reservoir.

In a further aspect the present invention provides for the use of calcium antagonists of the dihydropyridine type for the manufacture of a TTS for administration of calcium antagonists of the dihydropyridine type through a pre-determined area of intact skin for the treatment of cardiovascular disorders including hypertension.

In a preferred embodiment, the present invention provides a TTS for administering calcium antagonists of the dihydropyridine type, especially lacidipine or nifedipine, in the form of skin patch.

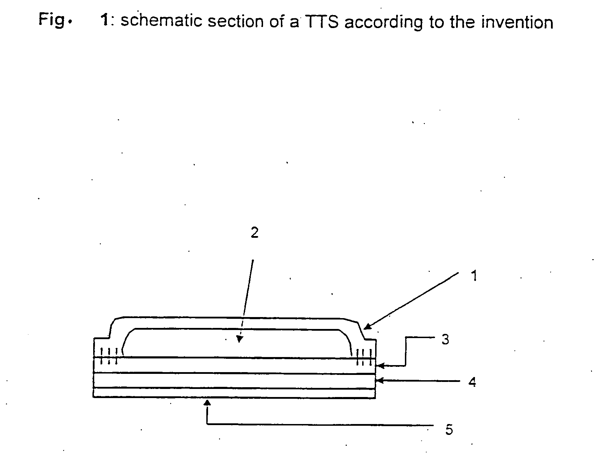

Figure 1 of the accompanying drawings gives a schematic section of a transdermal therapeutic system according to the invention.

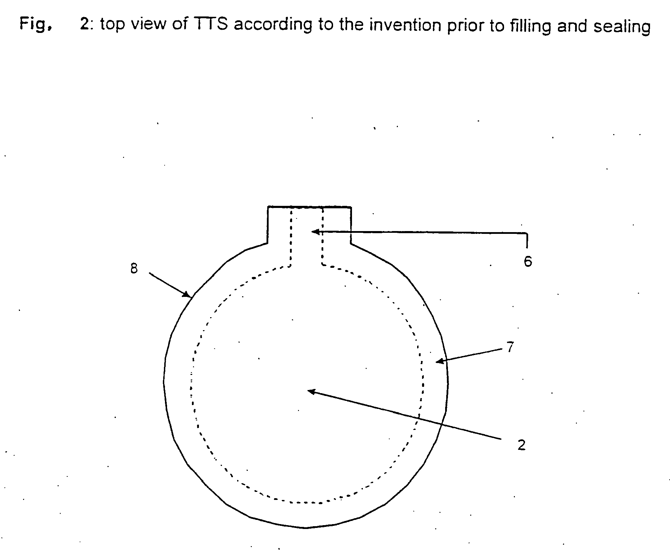

Figure 2 of the accompanying drawings gives a top view of a transdermal therapeutic system according to the invention prior to fill and sealing.

For a therapeutic transdermal system according to the invention the backing layer (1) is preferably made of a sheet or a film of a flexible material that is substantially impermeable to the solution of the calcium antagonist of the dihydropyridine type. The layer is preferably of the order of 50 - 200 µm in thickness and may be optionally pigmented. Conveniently the backing layer (1) is heat sealable to the control membrane (3).

The layer (I) is preferably of a material that permits the device to follow the contours of the skin and be worn comfortably on areas of the skin such as joints of flexure. Examples of flexible polymers useful for the backing layer include polyethylene, polypropylene, polyesters and the like, which may be provided as films or laminates. A preferred flexible polymer is a laminate consisting of pigmented polyethylene aluminium vapour coated polyester and a medium density polyethylene or ethylene vinyl acetate heat seal layer available from 3M™ under the trade mark Scotchpack ™1006.

The solution comprising a calcium antagonist of the dihydropyridine type and at least one skin permeation enhancer may be in a liquid, semisolid or thixotropic form and is contained within the drug reservoir (2).

A suitable amount of a calcium antagonist of the dihydropyridine type present in the solution is within the range 1 - 20 % e.g. 1 - 10 % by weight of the total solution.

Examples of suitable solvents for preparing the solution of a calcium antagonist of the dihydropyridine type include an alkanol e.g. ethanol, propanol or isopropanol or N-methyl-2-pyrrolidinone or mixtures thereof e.g. ethanol and N-methyl-2-pyrrolidinone.

Example of suitable skin permeation enhancers of this invention include saturated and unsaturated fatty acid esters, alcohols such as ethanol, propanol, isopropanol, n-decyl alcohol, etc, pyrrolidone derivatives (i.e. N-methyl-2- pyrrolidone) or (+)1-methyl-4-(1-methylethenyl)cyclohexene: ((+) limonene).

Conveniently fatty acid ester enhancers include esters of carboxylic acids containing from C₈ to C₁₆ carbon atoms. Preferred are those esters derived from palmitic acid, steric acid or lauric acid.
Conveniently fatty acid esters for use in the invention include fatty acid esters polyhydroxy alcohols such as sorbitol, glycerol or propylenglycol. Particularly preferred are fatty acids esters include those derived from sorbitol and of those sorbitan palmitate (Span™40) is particularly preferred.

Use of combinations of two or more of the skin permeation enhancer compounds may frequently result in superior results, such as greater transdermal absorption. Thus it has been found that a mixture of ethanol, N-methyl-2-pyrrolidone and sorbitan palmitate (Span™ 40) is a preferred skin permeation enhancing mixture.

The amount of ethanol present is conveniently within the range 10 - 60 % e.g. 30-40 % by weight of the total reservoir solution. The amount of Span™ 40 is conveniently within the range 0.5 - 6.0 % e.g. 1 - 5 % of the total reservoir solution The amount of N-methyl-2-pyrrolidone present is conveniently within the range 20 - 70 % e.g. 40 - 70 % by weight of the total reservoir solution.

A particularly preferred reservoir solution of the invention contains 3 - 5 % e.g. 4 % of a calcium antagonist of the dihydropyridine type, such as lacidipine, 30 - 40 % e.g. 36.5 % of ethanol, 3 to 5 % e.g. 3.5 % of Span™ 40, and 50 - 60 % e.g. 56 % of N-methyl-2-pyrrolidone by weight of the total solution.

The solution comprising a calcium antagonist of the dihydropyridine type with one or more skin permeation enhancers forms a further aspect of the invention. This solution may be prepared by dissolving the calcium antagonist of the dihydropyridine type in a solution of the enhancers and the solvents using conventional procedures.

The membrane (3) to control the release of the calcium antagonist of the dihydropyridine type is a thin, flexible uniformly microporous, flat sheet membrane which provides a constant rate of drug release independent of time or of the amount of the active ingredient that remains in the reservoir. A preferred membrane is a flat sheet membrane made from food grade polypropylene and polyethylene resins known under the Trade Mark Celgard™ 2400 or Celgard™ 2500, available from Hoechst Celanese. Celgard™ 2400 is the preferred membrane. Other suitable membranes include a microporus polyethylene membrane Solupor™ or an EVA membrane e.g. Co Tran™.

The contact adhesive layer (4) is a pressure-sensitive adhesive suitable for long term skin contact. It must also be physically and chemically compatible with the calcium antagonist of the dihydropyridine type and the vehicles employed. Further active ingredients must be soluble in the adhesive, so that the drug does not partition into the backing layer, but will partition into the skin. Conveniently the contact adhesive layer also adheres to the membrane (3).

Suitable adhesives include silicones, polyisobutylenes, polyacrilates, polyuretanes, plasticized ethylene, vinylacetate co-polymers, polystyrene-isoprene copolymer and a mixture thereof. Presently preferred contact adhesives are polyacrylates, silicones and polyurethanes.

Particularly preferred are the amine resistant silicone based pressure sensitive adhesives such as BIO-PSA Q7-4301, available from the Dow Corning Corp.

The release liner (5) is a disposable element which serves only to protect the adhesive layer prior to application to the skin. Typically, the release liner is formed from a material impermeable to the drug, vehicle, and adhesives and which is easily stripped from the contact adhesive.

Release liners are typically treated with silicone or fluorocarbons. A fluoro coated polyester film under the Trade Mark Scotchpatch™ 1022 available from 3M is particularly preferred.

In a further aspect of the invention provides a method for administering a calcium antagonist of the dihydropyridine type to a pre-determined area of intact skin, over defined time period and at an administration rate to reach and maintain an effective therapeutic dose of the calcium antagonist of the dihydropyridine type for the control of hypertension and other cardiovascular diseases. In order to reach the effective blood levels of the drug a preferred rate of administration is between 0.1 to 2 µg/hr, more preferably in the range of 0.4 to 0.6 µg/hr, through a skin area of 2.0 to 90 cm², more preferably 10 to 40 cm². The amount of the drug delivered into the skin may be controlled by a number of factors, including skin patch size, degree of initial drug loading, the choice of skin permeation enhancers and the control release membrane.

The efficacy of the transdermal therapeutic system to deliver the calcium antagonist of the dihydropyridine type at the required rate and over the required time scale can be determined using conventional in vitro and in vivo test procedures. Thus for example using the in vitro procedure that is described by Franz J. T. Journal of Investigative Dermatology 64(3) 190 - 5 1975.

The present invention also provides a process for the production of the transdermal therapeutic system according to the invention which comprises the following steps:
a) coating the release liner (5) with the adhesive layer (4) which is then laminated with the control membrane (3);
b) securing the backing layer (1) to the control membrane (3) by means of a seal (7) so as to obtain the sachet (8) having an opening (6);
c) filling the reservoir (2) in the sachet (8) via the opening (6) with a solution comprising a calcium antagonist of the dihydropyridine type and at least one skin permeation enhancer and then sealing the opening (6);

In the preparation of the open reservoir sachet (8) it is convenient to use the backing layer (1) and the laminate comprising members (3), (4) and (5) in sheet form and when the said backing layer is sealed to the said laminate then the sachet (8) of the desired size and shape can be stamped or punched out either simultaneously with its formation or in a subsequent operation.

The individual TTS can be sealed into an appropriate packaging material using standard methods in the art. A convenient packaging material for use comprises a laminate of paper, polymer (i.e. polyethylene) and aluminium film. An example of a suitable means to seal the individual TTS into the appropriate packaging material is a polyethylene polymer available from Du Pont and known under Trade Mark Surlyn™ The example presented below serves to illustrate the invention.

### Example 1

### a) Preparation of the reservoir solution containing lacidipine - Dose per patch

N-methyl pyrrolidone(1.12 g) and sorbitan palmitate (Span™ 40) (0.07 g) were added to ethanol (0.737 g) and the solution obtained was stirred for about 30 min. Lacidipine (80 mg) was then added under stirring to obtain a homogeneous solution.

### b) Preparation of the Transdermal Therapeutic System (TTS)

A solution of the silicone adhesive (4) [BIO-PSA Q7-4301: silicone resin, amine resistant, high tack 200 g/cm²] was coated onto the release liner (5) [Scotchpak® 1022]. The control membrane (3) (Celgard® 2400) was then laminated to the dried adhesive layer. The backing layer (1) (Scotchpak® 1006) was then secured to the control membrane with a heat seal (7) to form a sachet (8) having a drug reservoir (2) connected to an opening (6). The drug reservoir (2) is then filled with the solution comprising lacidipine and at least one skin permeation enhancer via the opening (6) which is then heat sealed.

The patches of the following examples were prepared in an analogous manner

### Example 2

### Preparation of the reservoir solution containing nifedipine- Dose per patch

(+)-Limonene (0.37 g) was added to ethanol (1.60 g) and the solution obtained was stirred. Nifedipine (36 mg) was then added under stirring to obtain a homogeneous solution.

### Example 3

### Preparation of the reservoir solution containing nifedipine- Dose per patch

N-methyl pyrrolidone(1.12 g) and sorbitan palmitate (Span™ 40) (0.07 g) were added to ethanol (0.737 g) and the solution obtained was stirred for about 30 min. Nifedipine (82 mg) was then added under stirring to obtain a homogeneous solution.

## Claims

1. A transdermal therapeutic system for administering a calcium antagonist of the dihydropyridine type which comprises
(a) a backing layer, which defines the upper surface of the device,
(b) a drug reservoir containing a solution comprising
- a calcium antagonist of the dihydropyridine type,
- an alcohol selected from the group consisting of ethanol, propanol, isopropanol and n-decyl alcohol,
- a pyrrolidone derivative, and
- a saturated or unsaturated fatty acid ester of a carboxylic acid containing 8-16 carbon atoms and a polyhydroxy alcohol,
(c) a membrane to control the release of the active ingredient, and
(d) a pressure sensitive adhesive layer for attaching the system to the skin and, if necessary, a release liner on the outer face of the adhesive layer wherein the said backing layer and said membrane are connected together to form the drug reservoir.

2. A transdermal therapeutic system as claimed in claim 1 wherein the solution in the drug reservoir comprises a calcium antagonist of the dihydropyridine type, ethanol, N-methyl-2-pyrrolidinone and sorbitan palminate.

3. A transdermal therapeutic system as claimed in claim 2 wherein the solution comprises a calcium antagonist of the dihydropyridine type 3 - 5 %, ethanol 30 - 40 %, sorbitan palmitate 3 - 5 % and N-methyl-2-pyrrolidinone 50 - 60 % by weight of the total solution.

4. A transdermal therapeutic system as claimed in any of claims 1 to 3 in the form of skin patch.

5. A transdermal therapeutic system as claimed in any of claims 1 to 4 in which the calcium antagonist of the dihydropyridine type is selected from the group consisting of amlodipine, felodipine, isradipine, lacidipine, nicardipine, nifedipine, nilvadipine, nimodipine, nisoldipine, and nitrendipine.

6. A transdermal therapeutic system as claimed in any of claims 1 to 5 in which the calcium antagonist of the dihydropyridine type is lacidipine.

7. A transdermal therapeutic system as claimed in any of claims 1 to 5 in which the calcium antagonist of the dihydropyridine type is nifedipine..

8. The use of a calcium antagonist of the dihydropyridine type for the manufacture of a transdermal therapeutic system as claimed in any of claims 1 to 7 for administration of a calcium antagonist of the dihydropyridine type through a pre-determined area of intact skin for the treatment of cardiovascular disorders including hypertension.

9. A solution which is suitable for use in a transdermal therapeutic system as claimed in any of claims 1 to 7 which comprises a calcium antagonist of the dihydropyridine type, ethanol, N-methyl-2-pyrrolidinone and sorbitan palmitate.

## Patentansprüche

1. Transdermales therapeutisches System zur Verabreichung eines Calciumantagonisten des Dihydropyridintyps, umfassend
a) eine Unterlage, die die obere Oberfläche der Vorrichtung definiert,
b) ein Arzneimittelreservoir, enthaltend eine Lösung, umfassend
- einen Calciumantagonisten des Dihydropyridintyps,
- einen Alkohol, ausgewählt aus der aus Ethanol, Propanol, Isopropanol und n-Decylalkohol bestehenden Gruppe,
- ein Pyrrolidonderivat und
- einen gesättigten oder ungesättigten Fettsäureester aus einer Carbonsäure mit 8-16 Kohlenstoffatomen und einem Polyhydroxyalkohol,
c) eine Membran, die die Freisetzung des Wirkstoffs steuert, und
d) eine druckempfindliche Klebeschicht zum Befestigen des Systems auf der Haut und, falls erforderlich, eine wiederablösbare Schutzschicht auf der äußeren Fläche der Klebeschicht, wobei die Unterlage und die Membran unter Bildung des Arzneimittelreservoirs miteinander verbunden sind.

2. Transdermales therapeutisches System nach Anspruch 1, wobei die Lösung in dem Arzneimittelreservoir einen Calciumantagonisten des Dihydropyridintyps, Ethanol, N-Methyl-2-pyrrolidinon und Sorbitanpalmitat enthält.

3. Transdermales therapeutisches System nach Anspruch 2, wobei die Lösung, bezogen auf die gesamte Lösung, 3-5 Gew.-% eines Calciumantagonisten des Dihydropyridintyps, 30-40 Gew.-% Ethanol, 3-5 Gew.-% Sorbitanpalmitat und 50-60 Gew.-% N-Methyl-2-pyrrolidinon enthält.

4. Transdermales therapeutisches System nach einem der Ansprüche 1 bis 3 in Form eines Hautpflasters.

5. Transdermales therapeutisches System nach einem der Ansprüche 1 bis 4, wobei der Calciumantagonist des Dihydropyridintyps aus der aus Amlodipin, Felodipin, Isradipin, Lacidipin, Nicardipin, Nifedipin, Nilvadipin, Nimodipin, Nisoldipin und Nitrendipin bestehenden Gruppe ausgewählt ist.

6. Transdermales therapeutisches System nach einem der Ansprüche 1 bis 5, wobei es sich bei dem Calciumantagonisten des Dihydropyridintyps um Lacidipin handelt.

7. Transdermales therapeutisches System nach einem der Ansprüche 1 bis 5, wobei es sich bei dem Calciumantagonisten des Dihydropyridintyps um Nifedipin handelt.

8. Verwendung eines Calciumantagonisten des Dihydropyridintyps zur Herstellung eines transdermalen therapeutischen Systems nach einem der Ansprüche 1 bis 7 zur Verabreichung eines Calciumantagonisten des Dihydropyridintyps über eine zuvor festgelegte Fläche intakter Haut zur Behandlung von Herzkreislauferkrankungen einschließlich Bluthochdruck.

9. Lösung, die sich zur Verwendung in einem transdermalen therapeutischen System nach einem der Ansprüche 1 bis 7 eignet und einen Calciumantagonisten des Dihydropyridintyps, Ethanol, N-Methyl-2-pyrrolidinon und Sorbitanpalmitat enthält.

## Revendications

1. Système thérapeutique transcutané pour l'administration d'un antagoniste de calcium de type dihydropyridine comprenant
(a) une couche substrat qui définit la surface supérieure du dispositif,
(b) un réservoir de médicament contenant une solution comprenant
- un antagoniste de calcium de type dihydropyridine,
- un alcool pris dans le groupe constitué par l'éthanol, le propanol, l'isopropanol et l'alcool n-décylique,
- un dérivé de la pyrrolidone, et
- un ester d'acide gras saturé ou insaturé d'un acide carboxylique présentant 8 à 16 atomes de carbone et un alcool polyhydroxylique,
(c) une membrane pour contrôler la libération de l'ingrédient actif, et
(d) une couche adhésive sensible à la pression pour fixer le système à la peau et, si nécessaire, une pochette de délivrance sur la face externe de la couche adhésive, où ladite couche substrat et ladite membrane sont reliées en vue de former le réservoir de médicament.

2. Système thérapeutique transcutané tel que revendiqué dans la revendication 1, où la solution dans le réservoir de médicament comprend un antagoniste de calcium de type dihydropyridine, l'éthanol, la N-méthyl-2-pyrrolidinone et le palmitate du sorbitane.

3. Système thérapeutique transcutané tel que revendiqué dans la revendication 2, où la solution comprend un antagoniste de calcium de type dihydropyridine 3-5 %, l'éthanol 30-40 %, le palmitate de sorbitane 3-5 % et la N-méthyl-2-pyrrolidinone 50-60 % en masse de la solution totale.

4. Système thérapeutique transcutané tel que revendiqué dans l'une quelconque des revendications 1 à 3 sous forme d'un timbre dermique.

5. Système thérapeutique transcutané tel que revendiqué dans l'une quelconque des revendications 1 à 4, dans lequel l'antagoniste de calcium de type dihydropyridine est pris dans le groupe constitué par l'amlodipine, la felodipine, l'isradipine, la lacidipine, la nicardipine, la nifédipine, la nilvadipine, la nimodipine, la nisoldipine et la nitrendipine.

6. Système thérapeutique transcutané tel que revendiqué dans l'une quelconque des revendications 1 à 5, dans lequel l'antagoniste de calcium de type dihydropyridine est la lacidipine.

7. Système thérapeutique transcutané tel que revendiqué dans l'une quelconque des revendications 1 à 5, dans lequel l'antagoniste de calcium de type dihydropyridine est la nifédipine.

8. Utilisation d'un antagoniste de calcium de type dihydropyridine pour la fabrication d'un système thérapeutique transcutané tel que revendiqué dans l'une quelconque des revendications 1 à 7, pour l'administration d'un antagoniste de calcium de type dihydropyridine à travers une aire pré-déterminée de peau intacte pour le traitement de troubles cardiovasculaires y compris l'hypertension.

9. Solution qui est appropriée pour l'utilisation dans un système thérapeutique transcutané tel que revendiqué dans l'une quelconque , des revendications 1 à 7, comprenant un antagoniste de calcium de type dihydropyridine, l'éthanol, la N-méthyl-2-pyrrolidinone et le palmitate de sorbitane.
